# EUROPEAN PATENT SPECIFICATION

(11) **EP 0 922 695 B1**
(45) Date of publication and mention of the grant of the patent: **27.11.2002**
(21) Application number: 98310193.2
(22) Date of filing: 11.12.1998
(51) Int. Cl.: C07C 303/32, C07C 309/42, C07C 67/08, C07C 67/14, C07F 9/12

(54) **Preparation of esters**
Herstellung von Carbonsäureestern
Préparation d'esters

(30) Priority: 11.12.1997 GB 9726246
(43) Date of publication of application: 16.06.1999
(60) Divisional application: 01121707.2
(73) Proprietor: THE ASSOCIATED OCTEL COMPANY LIMITED, London W1X 6DT (GB)
(72) Inventor: Radley, P. M., The Associated Octel Company Ltd, Ellesmere Port, South Wirral L65 3HF (GB); Head, R. A., The Associated Octel Company Ltd, Ellesmere Port, South Wirral L65 3HF (GB); Small, Andrew, The Associated Octel Company Ltd, Ellesmere Port, South Wirral L65 3HF (GB)
(74) Representative: Matthews, Derek Peter

(56) References cited:
- EP-A- 0 153 222
- EP-A- 0 270 304
- EP-A- 0 294 073
- EP-A- 0 402 048
- US-A- 5 523 434

## Description

The invention relates to an improved process for the preparation of phenol esters which have utility as bleach activators.

As a result of renewed interest in the use of phenolsulphonate esters as bleach activators, there has been a resurgence of interest in improved methods of producing them.

EP 148148 describes the reaction of anhydrous sodium phenol sulphonate with an alkanoyl halide at 90-200°C in the absence of a solvent.

EP 120591 describes a similar reaction but uses refluxing halocarbon solvents as the reaction medium. Additionally, 1.2-1.5 mole equivalents of alkanoyl halide are employed as are extended reaction times (20 hours) to effect the reaction.

EP 150532 describes the reaction of an acyl chloride with anhydrous disodium phenol sulphonate (^{∼}1:1 mole ratio) in glyme as a solvent. Typical reaction yields are 60-80%.

EP 166571 describes a similar process but uses a 1.2-1.5 mole equivalent of acyl chloride to give products in isolated yields of 40-65%.

Improved yields are claimed in EP 220826 wherein certain linear acyl chlorides are reacted with sodium phenol sulphonate at 160-170°C over 3-4 hours. A 2:1 mole ratio is typically employed and the necessary recycle of unreacted acid chloride is demonstrated.

Process improvements are also claimed in EP 402048 wherein a phase transfer catalyst is used to effect the reaction of an acid chloride with a phenol sulphonate salt. Isolated yields are typically 60-70% based on the starting acid chloride employed.

EP 164786 describes the use of potassium salts of phenolsulphonic acid instead of the sodium salt to improve reaction yields although no quantitative data is provided.

The prior art discussed above clearly demonstrates that obtaining conversion factors greater than 70% using acyl chlorides and salts of phenol sulphonic acid is difficult to achieve. Thus, a number of alternative processes have also been taught.

EP 125641 teaches the use of transesterification to effect the desired reaction whereby phenol esters of aliphatic acids are reacted with hydroxybenzene sulphonate salts. Very large molar excesses of phenol esters are used (up to 5:1) resulting in isolated yields of only 20-30% based on the starting phenol ester employed.

US 4544503 describes the reaction of phenol sulphonate salts with CO and an olefin at >125°C and >500 psig in an inert atmosphere with a metal carbonyl catalyst. Moderate yields are given (^{∼}60%), and products are highly coloured.

Sulphonation of an appropriate phenol ester is described in EP 165480 and EP 201222 wherein good yields (70-90%) are claimed by careful control of the SO₃ sulphonation conditions.

Alternatively, the use of a mixed anhydride using acetic anhydride is described in US 4735740.

Similarly, the preparation of 6-benzoylamidocaproyl oxybenzene sulphonate is described in US 4634551 whereby the acid is reacted with anhydrous sodium phenol sulphonate using trifluoroacetic anhydride. Isolated yield was 48%.

EP 153222 describes the use of a symmetric anhydride to effect the reaction, acetic anhydride is disclosed but this process suffers from the principal disadvantage of only giving a maximum yield of 50% based on the starting acid on a first-pass process. Recycling is necessary and this is described.

Finally, the use of the Schotten-Baumann technique in the preparation of esters of oxybenzene sulphonate salts is given in EP 294073. The only synthesis exemplified involves the reaction of sodium phenol sulphonate with benzoyl chloride to make sodium benzoyloxybenzene sulphonate. No quantitative data is provided.

Summarising these disclosures, it can be seen that, despite considerable prior art teaching, there remain significant problems in finding simple, high-yielding, low colour generating, cost-effective processes to make oxybenzene salts such as oxybenzene sulphonate salts.

We have now developed improved processes for the preparation of phenol esters of formula (I) 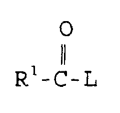 wherein R¹ is a linear or branched alkyl or alkenyl group of 3 to 20 carbon atoms optionally interrupted by -O- or -NH- and optionally substituted by an oxo group and L is a group of formula 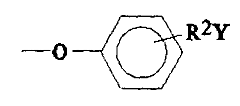 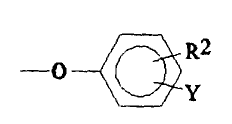 or 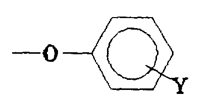 in which R² is an alkyl group or alkylene chain containing 1 to 8 carbon atoms and Y is a group -CO₂^{⊖}M^{⊕}, -SO₃^{⊖}M^{⊕} or -PO₄^{⊖}M^{⊕} in which M^{⊕} is a cation, preferably an alkali metal, ammonium or substituted ammonium cation. In particular M^{⊕} may be a sodium or potassium cation. It will be appreciated that the -O- or -NH- in R¹ can be adjacent to the oxo substituent thus forming an ester or amide linkage.

In a first aspect of the invention a carboxylic acid corresponding to R¹-CO- as defined above is reacted with a substituted phenol corresponding to L as defined above in the presence of a chlorinating agent such as thionyl chloride, phosgene, POCl₃ or PCl₅. At the end of the reaction the desired phenol ester, formed in high yield, is obtained by filtration. This then comprises a "one pot" synthesis of phenol ester from carboxylic acid.

The term "chlorinating agent" as used above means an agent capable of forming an acyl chloride from a carboxylic acid.

Suitable solvents for use in the invention are aromatic hydrocarbons, cycloalkanes, dialkylamides, cyclic dialkylamides, ethers, alkyl nitriles, dialkylketones and esters.

Substituted phenols corresponding to L as defined above are commercially available from numerous suppliers. It is important that any water present is partly or fully removed before use. This can be achieved by drying or by azeotropic removal of the water in the presence of an appropriate solvent. In such cases for best results a reaction solvent should be selected which permits the azeotropic removal of water prior to the addition of the chlorinating agent. Examples of such solvents include aliphatic and aromatic hydrocarbons, ketones, cyanoalkanes and dialkylamides, the selection of which is well within the knowledge of those skilled in the art.

Suitable reaction temperatures for the "one pot" reaction are -20°C to 250°C. Preferably the "one pot" reaction is carried out above 60°C to allow HCl and SO₂ to off-gas.

Reaction time for the reaction is variable to completion (dependent upon solvent system and temperature). Generally the reaction time is less than 5 hours and more than 1 hour.

The colour of the solid product is a commercially important attribute. Material of a white appearance is desired. The colour of the product can be quantified in terms of Hunter colour values L, a and b, where colour improvement is shown by an increase in L and a decrease in a and b. The colour characteristics of product from this process can be improved by addition of a polar solvent to the final reaction mixture. A suitable solvent is acetone. Suitable quantities will need to be determined on a case-by-case basis, but will generally lie in the range 5-50% by volume, relative to the reaction volume.

Whilst it is normally convenient to react the substituted phenol and the carboxylic acid and chlorinating agent in approximately equimolar amounts, it will occasionally be found that the reactions will not proceed to completion under these conditions, for example if the substituted phenol contains a small amount of residual water. An additional quantity of chlorinating agent may then be added to bring the reaction to completion.

The invention is particularly applicable to the synthesis of sodium (6-nonamidohexanoyl)oxybenzene sulphonate.

### Example 1

### One-pot preparation of sodium (6-nonamidohexanoyl)oxybenzene sulphonate

A slurry of 23.2g (0.10 mol) of sodium phenolsulphonate dihydrate and 250 ml of toluene was heated to reflux for three hours with azeotropic removal of a total of 30 ml of toluene and water using a Dean-Stark trap. The mixture was allowed to cool to 20°C and 27.1g (0.10 mol) of nonamidohexanoic acid added. The mixture was cooled to 0-5°C and 12.0g (0.10 mol) of thionyl chloride added. The mixture was gently heated to reflux for three hours and then allowed to cool to room temperature. The white solid product was collected by filtration, washed with 20 ml of toluene and dried at 40-50°C under reduced pressure to give the sodium (6-nonamidohexanoyl)-oxybenzene sulphonate, 44.1g (0.098 mol), 98% yield, 96.35% assay by HPLC.

### Example 2

### Use of acetone to improve colour characteristics

Two procedures identical to Example 1 were carried out. One was worked up as described in Example 1. To the other 100 ml of acetone were added at room temperature after the reaction phase had finished, followed by filtration as in Example 1. The characteristics of the products were:

| | No added acetone | Added acetone |
|---|---|---|
| Yield (%) | 93.1 | 99.6 |
| Assay (%) | 94 | 94 |
| Hunter L | 83.6 | 88.8 |
| a | 0.88 | 0.51 |
| b | 8.63 | 4.39 |

## Claims

1. A process for preparing a phenol ester of formula I 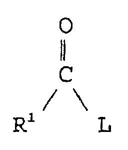 (wherein R¹ is a linear or branched alkyl or alkenyl group optionally interrupted by an -O- or -NH- group and optionally substituted by an oxo group and L is a group of formula 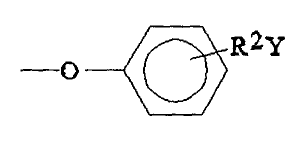 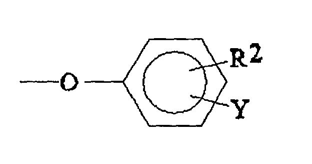 or 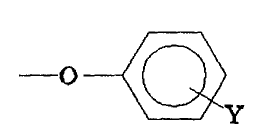 in which R² is an alkyl group or alkylene chain and Y is a group -CO₂⁻M⁺, SO₃⁻M⁺ or PO₄⁻M⁺ (wherein M is a cation)) comprising the step of reacting a substituted phenol corresponding to L with a carboxylic acid corresponding to R¹CO- in the presence of a chlorinating agent.

2. A process as claimed in claim 1 wherein said substituted phenol is subjected to removal of water prior to said reaction step.

3. A process as claimed in claim 2 wherein said removal of water is carried out azeotropically in an appropriate solvent.

4. A process as claimed in any preceding claim in which the said reaction step is carried out under reflux.

5. A process as claimed in claim 4 in which the said reaction step is carried out at a temperature above 60°C.

6. A process as claimed in any preceding claim wherein said chlorinating agent is SOCl₂.

7. A process as claimed in any preceding claim wherein R¹CO- is a 6-acylamidohexanoyl group.

8. A process as claimed in any preceding claim further comprising addition of a colour-improving amount of a polar solvent after said reaction step, and recovery of said phenol ester by filtration.

9. A process as claimed in claim 8 wherein said polar solvent is acetone.

10. A process as claimed in claim 1 in which an excess of chlorinating agent is used.

11. A process as claimed in claim 1 in which a quantity of chlorinating agent is added to bring the reaction to completion.

12. A process as claimed in any preceding claim wherein said compound of formula I is sodium 6-(nonamido-hexanoyl)-oxybenzene sulphonate.

## Patentansprüche

1. Verfahren zur Darstellung eines Phenolesters mit Formel I 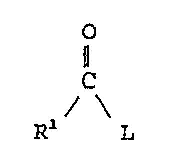 (wobei R¹ eine lineare oder verzweigte Alkyl- oder Alkenylgruppe ist, die wahlweise durch eine -O- oder -NH-Gruppe unterbrochen ist und wahlweise durch eine Oxogruppe substituiert ist, und L eine Gruppe mit Formel 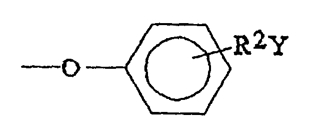 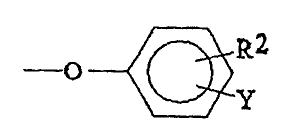 oder 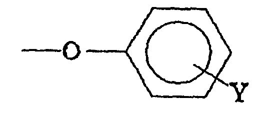 ist, wobei R² eine Alkylgruppe oder eine Alkylenkette ist und Y eine Gruppe -CO₂⁻M⁺, -SO₃⁻M⁺ oder -PO₄⁻M⁺ (wobei M ein Kation ist) ist, das die Schritte des Umsetzens eines L entsprechenden substituierten Phenols mit einer R¹CO- entsprechenden Carbonsäure in der Gegenwart eines Chlorierungsmittels umfaßt.

2. Verfahren nach Anspruch 1, wobei das substituierte Phenol vor dem Umsetzungsschritt einer Wasserentfernung unterzogen wird.

3. Verfahren nach Anspruch 2, wobei die Wasserentfernung azeotrop in einem geeigneten Lösungsmittel durchgeführt wird.

4. Verfahren nach einem der vorstehenden Ansprüche, bei dem der Umsetzungsschritt unter Rückfluß durchgeführt wird.

5. Verfahren nach Anspruch 4, bei dem der Umsetzungsschritt bei einer Temperatur von über 60 °C durchgeführt wird.

6. Verfahren nach einem der vorstehenden Ansprüche, wobei das Chlorierungsmittel SOCl₂ ist.

7. Verfahren nach einem der vorstehenden Ansprüche, wobei R¹CO- eine 6-Acylamidohexanoylgruppe ist.

8. Verfahren nach einem der vorstehenden Ansprüche, das weiterhin das Zugeben einer farbverbessernden Menge eines polaren Lösungsmittels nach dem Umsetzungsschritt und die Rückgewinnung des Phenolesters durch Filtration umfaßt.

9. Verfahren nach Anspruch 8, wobei das polare Lösungsmittel Aceton ist.

10. Verfahren nach Anspruch 1, wobei ein Überschuß des Chlorierungsmittels verwendet wird.

11. Verfahren nach Anspruch 1, bei dem eine Menge des Chlorierungsmittels zugegeben wird, um die Reaktion zur Vervollständigung zu bringen.

12. Verfahren nach einem der vorstehenden Ansprüche, wobei die Verbindung mit Formel I Natrium-6-(nonamidohexanoyl)-oxybenzensulfonat ist.

## Revendications

1. Procédé de préparation d'un ester de phénol de formule I 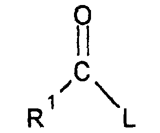 (dans laquelle R¹ est un groupe alkyle ou alcényle à chaîne droite ou ramifiée, éventuellement interrompu par un groupe -O- ou -NH-, et éventuellement substitué par un groupe oxo, et L est un groupe de formule 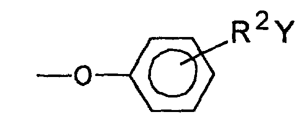 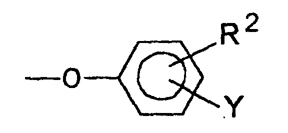 ou 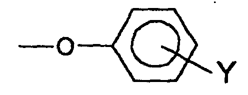 où R² est un groupe alkyle ou une chaîne alkylène, et Y est un groupe -CO₂⁻M⁺, SO₃⁻M⁺ ou PO₄⁻M⁺ (où M est un cation)),
qui comprend l'étape de réaction d'un phénol substitué correspondant à L avec un acide carboxylique correspondant à R¹CO-, en présence d'un agent de chloration.

2. Procédé selon la revendication 1, dans lequel ledit phénol substitué est soumis à une élimination d'eau avant ladite étape de réaction.

3. Procédé selon la revendication 2, dans lequel l'élimination d'eau est mise en oeuvre dans des conditions azéotropiques dans un solvant approprié.

4. Procédé selon l'une quelconque des revendications précédentes, dans lequel ladite étape de réaction est mise en oeuvre au reflux.

5. Procédé selon la revendication 4, dans lequel ladite étape de réaction est mise en oeuvre à une température supérieure à 60°C.

6. Procédé selon l'une quelconque des revendications précédentes, dans lequel ledit agent de chloration est le SOCl₂.

7. Procédé selon l'une quelconque des revendications précédentes, dans lequel R¹CO- est un groupe 6-acylamidohexanoyle.

8. Procédé selon l'une quelconque des revendications précédentes, qui comprend en outre l'addition d'une quantité apte à améliorer la couleur d'un solvant polaire après ladite étape de réaction, et la récupération dudit ester de phénol par filtration.

9. Procédé selon la revendication 8, dans lequel ledit solvant polaire est l'acétone.

10. Procédé selon la revendication 1, dans lequel on utilise un excès de l'agent de chloration.

11. Procédé selon la revendication 1, dans lequel on ajoute pour parachever la réaction une certaine quantité de l'agent de chloration.

12. Procédé selon l'une quelconque des revendications précédentes, dans lequel ledit composé de formule I est le 6-(nonamidohexanoyl)-oxybenzènesulfonate de sodium.
